# EUROPEAN PATENT SPECIFICATION

(11) **EP 4 007 560 B1**
(45) Date of publication and mention of the grant of the patent: **11.10.2023**
(21) Application number: 20742759.2
(22) Date of filing: 24.07.2020
(51) Int. Cl.: A61K 8/362, A61K 8/368, A61K 8/49, A61Q 5/00

(54) **AN ANTIDANDRUFF COMPOSITION**
ANTISCHUPPENZUSAMMENSETZUNG
COMPOSITION ANTIPELLICULAIRE

(30) Priority: 01.08.2019 EP 19189592
(43) Date of publication of application: 08.06.2022
(73) Proprietor: Unilever IP Holdings B.V., 3013 AL Rotterdam (NL); Unilever Global IP Limited, Wirral, Merseyside CH62 4ZD (GB)
(72) Inventor: KADAMKODE, Vinitha, Whitefield Bangalore 560 066 (IN); MITRA, Rupak, Whitefield Bangalore 560 066 (IN)
(74) Representative: Mathai, Neenu Grace
(86) International application number: PCT/EP2020/070905
(87) International publication number: WO 2021/018755

(56) References cited:
- EP-A2- 0 853 941
- CN-A- 106 565 986
- SOMAYEH GHAHARI ET AL: "Phytochemical screening and antimicrobial activities of the constituents isolated from Koelreuteria paniculata leaves", NATURAL PRODUCT RESEARCH, vol. 29, no. 19, 2 October 2015 (2015-10-02), pages 1865-1869, XP055625821, GB ISSN: 1478-6419, DOI: 10.1080/14786419.2015.1005617
- Nykaafrontendteam: "Dhathri Dheedhi Anti-Dandruff For Removing Dandruff Naturally Herbal Shampoo at Nykaa.com", , 18 June 2019 (2019-06-18), XP055626744, Retrieved from the Internet: URL:https://www.nykaa.com/dhathri-dheedhi- anti-dandruff-for-removing-dandruff-natura lly-herbal-shampoo-100ml/p/417720 [retrieved on 2019-09-27]
- PAN CHUN-XIU ET AL: "Investigation on the macromolecular network structure of Xianfeng lignite by a new two-step depolymerization", FUEL, IPC SCIENCE AND TECHNOLOGY PRESS, GUILDFORD, GB, vol. 109, 8 December 2012 (2012-12-08), pages 49-53, XP028557394, ISSN: 0016-2361, DOI: 10.1016/J.FUEL.2012.11.059
- Anonymous: "4-Oxovaleric acid - Surfactant - SAAPedia", , 8 September 2020 (2020-09-08), XP055728611, Retrieved from the Internet: URL:https://www.surfactant.top/en/saa/?typ e=detail&id=7524 [retrieved on 2020-09-08]
- Anonymous: "Anti-Dandruff (Coal Tar) Topical : Uses, Side Effects, Interactions, Pictures, Warnings & Dosing - WebMD", , 2 August 2017 (2017-08-02), XP055728624, Retrieved from the Internet: URL:https://web.archive.org/web/2017080201 3524/https://www.webmd.com/drugs/2/drug-15 1769/anti-dandruff-coal-tar-topical/detail s [retrieved on 2020-09-08]
- RYBICKI MACIEJ ET AL: "Molecular tracers preserved in Lower Jurassic "Blanowice brown coals" from southern Poland at the onset of coalification: Organic geochemical and petrological characteristics", ORGANIC GEOCHEMISTRY, vol. 102, 13 December 2016 (2016-12-13), pages 77-92, XP029818302, ISSN: 0146-6380, DOI: 10.1016/J.ORGGEOCHEM.2016.09.012

## Description

### Field of the invention

This invention relates to an antidandruff composition. The invention more particularly relates to a personal care or cleansing composition for hair and scalp comprising actives that interact to provide synergistic anti-dandruff benefits.

### Background of the Invention

The invention relates to an anti-dandruff composition useful for cleaning especially suitable for personal care and cleansing. More particularly, it relates to care or cleansing of the hair and scalp.

Hair care compositions generally provide cleansing or conditioning benefits or a combination of the two. Such compositions typically comprise one or more cleansing surfactants which generally aid in cleaning the hair and/or the scalp free of undesirable soil, particles, and fatty matter. Additionally, anti-dandruff benefit has been provided through hair care compositions. Dandruff is an issue that affects many people globally. The condition is manifested by the shedding of clumps of dead skin cells from the scalp. These are white in colour and provide an aesthetically displeasing appearance. A factor that contributes to dandruff are certain members of the Malassezia yeasts (a type of fungus). To combat these, anti-dandruff products have been developed in the form of hair cleansing shampoos. An example of a known anti-dandruff shampoo comprises sodium lauryl ether sulfate (an ethoxylated anionic surfactant) in combination with an anti-dandruff agent. Typical anti-dandruff agents used in hair care are metal pyrithione e.g zinc pyrithione (ZPTO), octopirox (piroctone olamine), azole antimicrobials (e.g. climbazole), selenium sulfide and combinations thereof.

The present inventors have been trying to develop new antidandruff actives or combinations of well know actives that combine synergistically to provide efficacy equal to or better than the above-mentioned actives. They tried various combinations of actives and finally hit up on a specific carboxylic acid with at least carboxyl groups viz. itaconic acid or an ester thereof in combination with benzoic acid or a salt thereof which at very specific ratio ranges surprisingly gives the desired antifungal efficacy thus displaying potential for antidandruff benefit.

Phytochemical screening and antimicrobial activities of the constituents isolated from Koelreuteria pciniculata leaves (SOMAYEH GHAHARI ET AL.) discloses Methanoiic extract of Golden rain leaves was fractionated by column chromatography on silica gel and 18 fractions were obtained. Antimicrobial activities of fractions were investigated against *Bacillus subiilis, Staphylococcus aureus, Escherichia coli* and *Pseudomonas aeruginosa* as quality control bacteria and fungus *Pyricularia grisea* which causes Blast disease in rice. Fractions showed more antibacterial activity at 0.04 g/ mL concentration only on *B*. *subiilis* and *S*. *aureus* as gram positive bacteria. Also, three fractions indicated excellent antifungal effect on fungus *P*. *grisea.*

Dhathri Dheedhi Anti-Dandruff For Removing Dandruff Naturally Herbal Shampoo (Dhathri Ayurveda PVT.LTD) is a shampoo products from an ayurvedic process suryapakavidhi with ingredients that have antimicrobial and anti-fungal properties to fight dandruff.

US20120101135 (Clariant International Ltd.)relates to liquid compositions which contain a) from 40 to 99.9% by weight of sorbitan monocaprylate and b) from 0.1 to 60% by weight of one or more antimicrobial substances selected from the group consisting of the components b1) to b5): b1) specific organic acids and the salts thereof, b2) specific formaldehyde donors, b3) specific isothiazolinones, b4) specific paraben esters and the salts thereof, and b5) specific pyridones and the salts thereof.

CN106565986 (ENPING HUI XIANG IND CO LTD) discloses a surface modified nano calcium carbonate, which comprises the following components in parts by weight: 500-550 parts of nano calcium carbonate base material, 4-5 parts of potassium pyrophosphate, 2-3 parts of itaconic acid, 5-6 parts of basic magnesium carbonate, 4-5 parts of anhydrous calcium chloride, 3-4 parts of phosphorus pentoxide, 2-4 parts of anhydrous sodium sulfite, 1-2 parts of amino acid, 2-3 parts of sulfamic acid, 2-4 parts of EDTA, 1-2 parts of alanine, 2-3 parts of phthalimide, 1-2 parts of sorbitol, 2-3 parts of sodium hexametaphosphate, 3-4 parts of sodium benzoate, 4-5 parts of dodecyl dimethyl betaine, 4-5 parts of dodecyl 2-hydroxyethyl betaine, 4-5 parts of lauramidopropyl betaine, 1-2 parts of proline, and 3-5 parts of APEG.

To the knowledge of the present inventors, an antimicrobial composition comprising the combination as claimed herein have not been known or published so far.

It is thus an object of the present invention to deliver antidandruff benefits from combination of well known materials.

### Summary of the Invention

In accordance with a first aspect is disclosed an antimicrobial composition being a shampoo or hair conditioner comprising itaconic acid or an ester thereof and benzoic acid or a salt thereof; and a cosmetically acceptable carrier comprising water and surfactant; wherein the weight ratio of the itaconic acid or ester thereof and benzoic acid or salt thereof is in the range of 1:40 to 40:1; and wherein the ester of itaconic acid is dimethyl itaconate Yet another aspect of the present invention relates to a composition comprising itaconic acid or an ester thereof and benzoic acid or a salt thereof; and a cosmetically acceptable carrier comprising water and surfactant; wherein the weight ratio of the itaconic acid or ester thereof and benzoic acid or salt thereof is in the range of 1:40 to 40:1 for use in combating dandruff; and wherein the ester of itaconic acid is dimethyl itaconate.

### Detailed Description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. In other words, in specifying any ranges of values, any particular upper value can be associated with any particular lower value.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

By 'An antidandruff composition' as used herein, is meant to include a composition for topical application to skin, hair and/or scalp of mammals, especially humans to eliminate or minimize the population of microorganisms thereon, preferably fungi that are implicated in dandruff. Such a composition is generally applied on to the desired topical surface of the body and then preferably rinsed off, in a few minutes thereafter. It includes any product applied to a human body for also improving appearance, cleansing, odor control or general aesthetics. The composition of the present invention is most preferably a shampoo in the conventional lotion or semi-liquid form.

According to a first aspect is disclosed an antidandruff composition comprising a combination of two actives viz. itaconic acid or an ester thereof and benzoic acid or a salt thereof.

Itaconic acid is an organic, unsaturated dicarboxylic acid. It is methacrylic acid in which one of the methyl hydrogens is substituted by a carboxylic acid group. The other names of itaconic acid are 2-Methylenesuccinic, Methylenebutanedioic acid. The molecular weight of itaconic acid is 130.1 g/mol. It is available as a dry white colored powder. The solubility in water is 1g/12ml. Itaconic acid is naturally produced by various fungi like Aspergillus *terreus, Ustilago zeae,* and *Ustilago maydis* and the yeast *Candida* sp. Aspergillus terreus is used for the industrial production of itaconic acid. Polymers of itaconic acid are widely used in cleaning and hygiene, energy and industry, surface coatings and agriculture. It is widely used in hair conditioning gels and in odour control products.

Itaconic acid has the following structure:

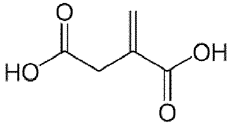

Itaconic acid esters may also be used in the composition of the invention. Suitable itaconic acid esters are dimethyl itaconate or dibutyl itaconate, preferably dimethyl itaconate.

The composition of the invention comprises benzoic acid or a salt thereof. Suitable salt of benzoic acid that may be included is sodium benzoate.

Benzoic acid is a compound comprising a benzene ring core carrying a carboxylic acid substituent. It has a role as an antimicrobial food preservative. Benzoic acid is available as a white crystalline solid. It is slightly soluble in water. Commercial benzoic acid is produced by the reaction of toluene with oxygen at temperatures around 200 °C in the liquid phase and in the presence of cobalt and manganese salts as catalysts. Acidic food and beverage like fruit juice (citric acid), sparkling drinks (carbon dioxide), soft drinks (phosphoric acid), pickles (vinegar) or other acidified food are preserved with benzoic acid. The structure of benzoic acid is:

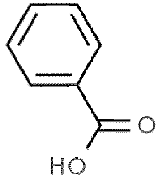

The weight ratio of itaconic acid or ester thereof to benzoic acid or a salt thereof is preferably in the range of 1:9 to 9:1.

The concentration of itaconic acid or ester thereof to enable the antidandruff efficacy is preferably in the range of 0.01 to 2%, preferably 0.1 to 2% by weight of the composition.

It is preferred that the benzoic acid or salt thereof is included in the composition in the range of 0.01 to 2%, preferably 0.1 to 2% by weight of the composition. Preferably, the total amount of itaconic acid or an ester thereof and benzoic acid or salt thereof is included in the composition in the range of 0.15 to 3%, preferably 0.2 to 2% by weight of the composition.

The composition of the invention additionally comprises a cosmetically acceptable carrier. The carrier is preferably chosen such that the composition of the invention can be delivered for use as a personal cleansing composition. As per one aspect the cosmetically acceptable carrier is water or an aqueous solution. According to another preferred aspect, the carrier additionally comprises a surfactant. The cosmetically acceptable vehicle is such that the composition can be prepared as a shampoo, conditioner, body wash, hand wash or face wash product, cream, lotion, gel, powder, ointment, or a soap bar.

According to a further preferred aspect of the present invention, the composition is either a shampoo, a hair conditioner, or a body wash product, most preferably a shampoo or a hair conditioner composition. When so formulated, the composition additionally comprises a cosmetically acceptable carrier selected from an anionic, cationic or amphoteric or zwitterionic surfactant. As per an especially preferred aspect of the invention, the composition is a shampoo. The composition of the invention especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%, further more preferably from 3 to 16% by weight of the composition. Preferred alkyl sulfates are C8-18 alky sulfates, more preferably C12-18 alkyl sulfates, preferably in the form of a salt with a solubilising cation such as sodium, potassium, ammonium or substituted ammonium.

Preferred alkyl ether sulfates are those having the formula: RO(CH₂CH₂O)ₙSO₃M; wherein R is an alkyl or alkenyl having from 8 to 18 (preferably 12 to 18) carbon atoms; n is a number having an average value of greater than at least 0.5, preferably between 1 and 3, more preferably between 2 and 3; and M is a solubilising cation such as sodium, potassium, ammonium or substituted ammonium. An example is sodium lauryl ether sulfate (SLES). SLES having an average degree of ethoxylation of from 0.5 to 3, preferably 1 to 3 is especially preferred.

Shampoo compositions according to the invention may comprise one or more further anionic cleansing surfactants which are cosmetically acceptable and suitable for topical application to the hair.

A composition of the invention preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, preferably from 0.5 to 8 wt.%, more preferably from 1 to 5 wt.% of a betaine surfactant

To enhance deposition of actives from compositions of the invention especially shampoos, cationic polymers are generally included therein. In the present invention too, it is preferred that the composition additionally includes 0.01 to 2.0% of a cationic polymer. The cationic polymer is preferably guar hydroxypropyl trimonium chloride. Guar polymer predominantly contains galactomannan polymer chains. This polymer is available at various molecular weights and degree of cationic substitutions depending on how much the guar has been hydrolysed and cationised. The cationic polymer is preferably present in 0.04 to 0.5%, more preferably 0.08 to 0.25% by weight of the composition.

The pH of the composition is preferably equal to or higher than 4.0, more preferably in the range of 5.0 to 10.0.

Preferably the composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives.

Suspending agent, if included, will generally be present in a shampoo composition of the invention at levels of from 0.1 to 10%, preferably from 0.5 to 6%, more preferably from 0.5 to 4% by total weight of suspending agent based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The composition of the invention is preferably aqueous based. It preferably comprises high amounts of water preferably from 70 to 95% by weight of the composition.

When conditioning benefits are to be delivered through the composition of the invention the composition is called a hair conditioner. Typically, the most popular conditioning agents used in hair care compositions are water-insoluble oily materials such as mineral oils, naturally occurring oils such as triglycerides and silicone polymers. Conditioning benefit is achieved by the oily material being deposited onto the hair resulting in the formation of a film, which makes the hair easier to comb when wet and more manageable when dry. An especially useful conditioning agent is a silicone compound, preferably a non-volatile silicone compound. Advantageously compositions herein may include one or more silicones. The silicones are conditioning agents found in dispersed or suspended particulate form. They are intended to deposit onto hair remaining behind after rinsing of the hair with water. Suitable silicone oils may include polyalkyl siloxanes, polyaryl siloxanes, polyalkylaryl siloxanes, polyether siloxane copolymers and mixtures thereof. Amino silicones are often formulated with shampoo compositions. Amino silicones are silicones containing at least one primary amine, secondary amine, tertiary amine or a quaternary ammonium group. High molecular weight silicone gums can also be utilized. Another useful type are the crosslinked silicone elastomers such as Dimethicone/Vinyl/Dimethicone Crosspolymers (e.g. Dow Corning 9040 and 9041).

Amounts of the silicone in compositions where present may range from about 0.1 to about 10 wt.%, preferably from about 0.1 to about 8wt.%, more preferably from about 0.3 to about 5wt.% by weight of the hair care compositions.

The pH of the composition is preferably equal to or higher than 4.0, more preferably in the range of 5.0 to 7.0.

The hair conditioning composition usually comprises conditioning surfactants selected from cationic surfactants, used singly or in admixture. Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof.

The most preferred cationic surfactants for use in the composition are stearamidopropyl dimethylamine, behentrimonium chloride, or stearyl trimethyl ammonium chloride. In conditioners of the invention, the level of cationic surfactant will generally range from 0.1 % to 5%, preferably 0.5 to 2.5% by weight of the composition.

Hair conditioning compositions of the invention preferably may also additionally comprise a fatty alcohol. The combined use of fatty alcohols and cationic surfactants in conditioning compositions is believed to be especially advantageous, because this leads to the formation of a lamellar phase, in which the cationic surfactant is dispersed.

The level of fatty alcohol in conditioners of the invention will generally range from 0.5 to 10%, preferably from 0.1 % to 8%, more preferably from 0.2 % to 7 %, most preferably from 0.3 % to 6 % by weight of the composition. The weight ratio of cationic surfactant to fatty alcohol is suitably from 1:1 to 1:10, more preferably from 1:1.5 to 1:8, optimally from 1:2 to 1:5.

The composition of the invention may be used for skin care e.g. body, hand or face wash. The antimicrobial composition may further comprise a surfactant. The preferred surfactants are nonionic surfactants, such as C₈-C₂₂, preferably C₈-C₁₆ fatty alcohol ethoxylates, comprising between 1 and 8 ethylene oxide groups when the product is in the liquid form. The surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16.

Thus, in a highly preferred aspect, the compositions include the surfactant selected from the group of anionic surfactant, fatty acid amide, alkyl sulphate, linear alkyl benzene sulphonate, and combinations thereof.

When the surfactants are present, the antimicrobial composition preferably comprises 1 to 90% surfactant by weight of the composition.

When surfactant is used, a particularly preferred surfactant is soap. Soap is a suitable surfactant for personal washing applications of the antimicrobial composition of the invention. The soap is preferably C₈-C₂₄ soap, more preferably C₁₀-C₂₀ soap and most preferably C₁₂-C₁₈ soap. The cation of the soap can be alkali metal, alkaline earth metal or ammonium. Preferably, the cation of the soap is selected from sodium, potassium or ammonium. More preferably the cation of the soap is sodium or potassium.

A typical fatty acid blend consisted of 5 to 30% coconut fatty acids and 70 to 95% fatty acids by weight of soap. Fatty acids derived from other suitable oils/fats such as groundnut, soybean, tallow, palm, palm kernel, etc. may also be used in other desired proportions.

When present, the soap, of the present is preferably present in an amount of 1 to 90%, preferably from 10 to 85%, more preferably 25 to 75% by weight of the composition. Preferred compositions may include other known ingredients such as perfumes, pigments, preservatives, emollients, sunscreens, emulsifiers, gelling agents and thickening agents. Choice of these ingredients will largely depend on the format of the composition.

Water is a preferred carrier. When water is present, it is preferably present in at least 1%, more preferably at least 2%, furthermore preferably at least 5% by weight of the composition. When water is the carrier, a preferred liquid composition comprises 10 to 99.8% by weight water.

Also disclosed in accordance with this invention is non-therapeutic use of the composition for delivering anti-dandruff benefits. The following non-limiting examples further illustrate preferred embodiments of the invention. All percentages referred to in the examples and throughout this specification are based on total weight unless otherwise indicated.

### Examples

### Examples 1-6: Antifungal efficacy of compositions as per the invention: compositions comprising itaconic acid and sodium benzoate

The samples as given in Table - 1 were subjected to their anti fungal efficacy on *M*. *furfur* as described hereinafter.

### Antifungal activity:

The test organism used in the assay is Malassezia *furfur* 14521. The *M. furfur* were revived from glycerol stock in YPD agar plates (yeast extract peptone dextrose media) containing 1% corn oil. The composition of YPD is yeast extract: 10g/litre, dextrose 20g/litre, Peptone 20g/litre and agarose 0.15%. The plates were incubated at 30°C for 72 hours for forming fungal colonies. A suspension culture of M.furfur was made using the colonies from plates in YPD broth. The optical density of the suspension culture was adjusted to 0.6 using spectrophotometer. This suspension was diluted in the ratio of 1:10 using YPD broth and 100µl of this culture was added into each well of 96 well test plate. A stock solution of 4X strength of test ingredients (itaconic acid and sodium benzoate) was prepared for each of the concentrations tested eg for 0.125% test concentration of itaconic acid/sodium benzoate a stock of 0.5% was prepared in YPD broth. 50µl of each of the test ingredient was added to 100µl of M*.furfur* culture, making the final volume to 200µl. In wells containing only one test ingredient, (itaconic acid or sodium benzoate only) 50 µl of test ingredient was added to 100µl of fungal culture and then made up the volume to 200µl with YPD broth. The plates were incubated at 30°C for 72 hours. Post incubation 10µl of 1% resazurin was added to each well and incubated for 18 hours at 30°C. Resazurin (7-Hydroxy-3H-phenoxazin-3-one 10-oxide) is a blue dye which is used as an oxidation reduction indicator in cell viability assays. The dehydrogenase enzymes in live cells reduce Resazurin (blue and non-fluorescent) to form the red fluorescent dye resorufin. The wells containing viable cells appear in pink color and the wells with dead cells appear blue/purple. The anti-fungal efficacy of the ingredient or combination of ingredients is calculated based on the colour development.

### Evaluation of synergy using fractional inhibitory concentration (ΣFIC)

The combinations of test ingredients with synergistic anti-fungal benefits were evaluated using ΣFIC method. The ΣFIC of the combinations were calculated using the following formula
- ΣFIC of combination A+B = [Concentration of A]/MIC of A + [concentration of B]/MIC of B, where MIC is the Minimum inhibitory concentration (MIC) of ingredient. MIC of a test ingredient is the lowest concentration of the ingredient required to inhibit the growth of the test organism.

The MIC of the various actives used was determined to be as follows:
Itaconic acid: 0.25 wt%
Sodium Benzoate:0.5 wt%
Dimethyl itaconate:0.5 wt%

The ΣFIC values for the various combinations are given in Table - 1 below:

**Table 1**

| **Example** | **Itaconic acid (wt%)** | **Sodium benzoate (wt%)** | **Ratio of itaconic acid: sodium benzoate** | **∑FIC** |
|---|---|---|---|---|
| 1 | 0.125 | 0.03 | 4.2:1 | 0.56 |
| 2 | 0.125 | 0.06 | 2.1:1 | 0.62 |
| 3 | 0.125 | 0.125 | 1:1 | 0.75 |
| 4 | 0.125 | 0.25 | 1:2 | 1.0 |
| 5 | 0.06 | 0.25 | 1:4.2 | 0.74 |
| 6 | 0.06 | 0.125 | 1:2.1 | 0.49 |

The data in Table 1 above indicates that a combination of itaconic acid and sodium benzoate within the weight ratios claimed gives synergistic antifungal activity.

### Examples 7-14: Antifungal efficacy of compositions as per the invention: Compositions comprising dimethyl itaconate and sodium benzoate

The samples as given in Table 2 were subjected to their anti fungal efficacy on M. *furfur* as described herein above.

### Antifungal activity:

The ΣFIC values for the various combinations are given in Table 2 below:

**Table 2**

| **Example** | **Dimethyl Itaconate (wt%)** | **Sodium benzoate (wt%)** | **Ratio of dimethyl itaconate: sodium benzoate** | **∑FIC** |
|---|---|---|---|---|
| 1 | 0.06 | 0.25 | 1:4.2 | 0.62 |
| 2 | 0.125 | 0.25 | 1:2.1 | 0.75 |
| 3 | 0.25 | 0.25 | 1:1 | 1.0 |
| 4 | 0.125 | 0.125 | 1:1 | 0.50 |
| 5 | 0.25 | 0.125 | 2:1 | 0.75 |
| 6 | 0.125 | 0.06 | 2.1:1 | 0.37 |
| 7 | 0.25 | 0.06 | 4.2:1 | 0.62 |

The data in Table 2 above indicates that a combination of dimethyl itaconate and sodium benzoate within the weight ratios claimed gives synergistic antifungal activity.

## Claims

1. An antimicrobial composition comprising itaconic acid or an ester thereof and benzoic acid or a salt thereof; and a cosmetically acceptable carrier comprising water and a surfactant;
wherein the weight ratio of the itaconic acid or ester thereof and benzoic acid or salt thereof is in the range of 1:40 to 40:1; and
wherein the ester of itaconic acid is dimethyl itaconate;
wherein the composition is a shampoo or a hair conditioner.

2. A composition as claimed in any one of the preceding claims wherein the concentration of itaconic acid or ester thereof is in the range of 0.01 to 2.0% by weight of the composition.

3. A composition as claimed in any one of the preceding claims wherein the concentration of benzoic acid or salt thereof is in the range of 0.01 to 2.0% by weight of the composition.

4. A composition as claimed in any one of the preceding claims wherein the total amount of itaconic acid or an ester thereof and benzoic acid or salt thereof is in the range of 0.15 to 3%, preferably 0.2 to 2% by weight of the composition.

5. A composition as claimed in any one of the preceding claims wherein the surfactant is selected from an anionic, cationic, amphoteric or zwitterionic surfactant.

6. A composition comprising itaconic acid or an ester thereof and benzoic acid or a salt thereof; and a cosmetically acceptable carrier comprising water and surfactant; wherein the weight ratio of the itaconic acid or ester thereof and benzoic acid or salt thereof is in the range of 1:40 to 40:1 for use in combating dandruff; and wherein the ester of itaconic acid is dimethyl itaconate.

## Patentansprüche

1. Antimikrobielle Zusammensetzung, umfassend Itaconsäure oder einen Ester davon und Benzoesäure oder ein Salz davon und einen kosmetisch akzeptablen Träger, der Wasser und ein Tensid umfasst;
wobei das Gewichtsverhältnis der Itaconsäure oder des Esters davon und der Bezoesäure oder des Salzes davon in dem Bereich von 1:40 bis 40:1 liegt; und
wobei der Ester der Itaconsäure Dimethylitaconat ist;
wobei die Zusammensetzung ein Shampoo oder ein Haarspülmittel ist.

2. Zusammensetzung, wie in irgendeinem der vorhergehenden Ansprüche beansprucht, wobei die Konzentration der Itaconsäure oder Esters davon in dem Bereich von 0,01 bis 2,0 Gewichts-% der Zusammensetzung liegt.

3. Zusammensetzung, wie in irgendeinem der vorhergehenden Ansprüche beansprucht, wobei die Konzentration der Benzoesäure oder Salzes davon in dem Bereich von 0,01 bis 2,0 Gewichts-% der Zusammensetzung liegt.

4. Zusammensetzung, wie in irgendeinem der vorhergehenden Ansprüche beansprucht, wobei die gesamte Menge der Itaconsäure oder eines Esters davon und der Benzoesäure oder Salzes davon in dem Bereich von 0,15 bis 3 Gewichts-%, vorzugsweise von 0,2 bis 2 Gewichts-% der Zusammensetzung liegt.

5. Zusammensetzung, wie in irgendeinem der vorhergehenden Ansprüche beansprucht, wobei das Tensid aus anionischem, kationischem, amphoterem oder zwitterionischem Tensid ausgewählt ist.

6. Zusammensetzung, umfassend Itaconsäure oder einen Ester davon und Benzoesäure oder ein Salz davon und einen kosmetisch akzeptablen Träger, der Wasser und Tensid umfasst; wobei das Gewichtsverhältnis der Itaconsäure oder Esters davon und der Benzoesäure oder Salzes davon in dem Bereich von 1:40 bis 40:1 zur Anwendung bei der Bekämpfung von Schuppen liegt und wobei der Ester der Itaconsäure Dimethylitaconat ist.

## Revendications

1. Composition antimicrobienne comprenant de l'acide itaconique ou un ester de celui-ci et de l'acide benzoïque ou un sel de celui-ci ; et un support cosmétiquement acceptable comprenant de l'eau et un tensioactif ;
dans laquelle le rapport en masse de l'acide itaconique ou ester de celui-ci et d'acide benzoïque ou sel de celui-ci se trouve dans l'intervalle de 1:40 à 40:1 ; et
dans laquelle l'ester d'acide itaconique est l'itaconate de diméthyle ;
dans laquelle la composition est un shampoing ou un après-shampoing.

2. Composition selon l'une quelconque des revendications précédentes, dans laquelle la concentration d'acide itaconique ou ester de celui-ci se trouve dans l'intervalle de 0,01 à 2,0 % en masse de la composition.

3. Composition selon l'une quelconque des revendications précédentes, dans laquelle la concentration d'acide benzoïque ou sel de celui-ci se trouve dans l'intervalle de 0,01 à 2,0 % en masse de la composition.

4. Composition selon l'une quelconque des revendications précédentes, dans laquelle la quantité totale d'acide itaconique ou d'un ester de celui-ci et d'acide benzoïque ou d'un sel de celui-ci se trouve dans l'intervalle de 0,15 à 3 %, de préférence 0,2 à 2 % en masse de la composition.

5. Composition selon l'une quelconque des revendications précédentes, dans laquelle le tensioactif est choisi parmi un tensioactif anionique, cationique, amphotère ou zwitterionique.

6. Composition comprenant de l'acide itaconique ou un ester de celui-ci et de l'acide benzoïque ou un sel de celui-ci ; et un support cosmétiquement acceptable comprenant de l'eau et un tensioactif ; dans laquelle le rapport en masse de l'acide itaconique ou ester de celui-ci et d'acide benzoïque ou sel de celui-ci se trouve dans l'intervalle de 1:40 à 40:1 pour une utilisation dans la lutte contre les pellicules ; et dans laquelle l'ester d'acide itaconique est l'itaconate de diméthyle.
